# EUROPEAN PATENT APPLICATION

(11) **EP 4 624 564 A1**
(43) Date of publication of application: **01.10.2025**
(21) Application number: 24167272.4
(22) Date of filing: 28.03.2024
(51) Int. Cl.: C12M 3/00, C12M 1/12, C12M 3/06, C12M 1/26, C12M 1/42

(54) **DEVICE FOR GENERATING A SOLIDIFIED TISSUE SAMPLE**

(71) Applicant: mimiX Biotherapeutics SA, 2503 Biel / Bienne (CH)
(72) Inventor: THURNER, Marc, 2075 Wavre (CH); SCHEIDEGGER, Andreas, 3032 Hinterkappelen (CH)
(74) Representative: P&TS SA (AG, Ltd.)

(57) **Abstract**

The present invention concerns a receptacle (11) for forming a solidified biological tissue sample, the receptacle comprising a frame member (4) arranged around the periphery of a base member (3) such that said frame member (4) and said base member (3) can perform a translational movement in relation to one another, wherein the receptacle is designed to alter between a charge state and a discharge state. The receptacle may further comprise one or more interface members for operationally coupling the receptacle (11) to a vibration generating device. The invention also concerns a system comprising the receptacle (11) and a vibration generating device provided with a coupling interface, as well as a method for generating waves in a substance contained in said receptacle (11) and for generating a biological tissue construct using the receptacle (11) and/or said system.

## Description

### Technical domain

The present invention concerns the formation of a solidified tissue sample from a liquid or gel-like substance containing particles. The invention further concerns the use of wave-induced patterning for generating solid three-dimensional structures and /or layer architectures. The technology is particularly useful for patterning of solid particles, for example cells, microtissue, or particles such than active ingredients of microbial agents, fragments, in a liquid or gel-like substance.

### Related art

Tissue engineering stands at the forefront of medical science, offering promising avenues for regenerative medicine, drug discovery, and personalized healthcare solutions. Central to tissue engineering is the ability to mimic and manipulate cellular behaviors within controlled environments to regenerate or repair damaged tissues and organs.

Several approaches using liquid or gel-like carrier substrates which are provided with or naturally containing cells and/or microtissues, such as hydrogels, processed blood samples, or whole blood have been described to generate solidified biological tissue samples in vitro. The utilization of cells or microtissues within a carrier substrate provides a conducive environment for cellular adhesion, proliferation, and differentiation, facilitating the orchestrated development of functional tissues. Moreover, the incorporation of whole blood as a medium offers a physiologically relevant milieu, enhancing the mimicry of *in vivo* conditions and promoting more accurate physiological responses.

WO2023161769 for example describes the formation of a skin tissue graft using a whole blood sample. The blood sample is provided in a flat-based receptacle, subjected to sound-induced morphogenesis (SIM) and allowed to coagulate to result in a solidified tissue construct with spatially patterned cells. The solidified, in this case coagulated, tissue construct can then be removed from the receptacle and used as a tissue graft for example.

The removal if the tissue sample from the receptacle in which it solidified, is however extremely delicate and often results in damages to the solidified tissue sample. The solidified tissue, which is often soft or gel-like, tends to adhere to the base and the side walls of the receptacle in which it solidified, rendering the safe removal of the sample extremely difficult. Despite careful handling the sample is often damaged along its periphery or ruptures inwardly from its edge, thereby ruining it entirely.

There is therefore a need to find an equipment which is better adapted for generating solidified biological tissue samples, for example coagulated blood samples, which allows for an easy and safe removal of the sample from the device after solidification of the sample.

### Short disclosure of the invention

It is an aim of this invention to provide a solution which overcomes the shortcomings and limitations of the state of the art.

It is an aim of the present invention to provide an equipment for forming solidified biological tissue samples, which is robust and delivers reproducible results.

It is another aim of the present invention to provide an equipment for forming solidified biological tissue samples which permits the safe removal of the solidified sample for further use.

It is yet another aim of the present invention to provide for an equipment which simplifies the production of solidified biological tissue samples, thereby improving the scalability of the production process.

It is yet another aim of the present invention to provide an equipment reproducibly producing solidified biological tissue samples comprising regularly spaced biological cells or microtissues.

According to the invention, one or more of these aims are achieved by the object of the attached claims, and especially by its independent claims. Further preferred embodiments are provided in the dependent claims.

In particular, one or more of the above-stated aims are achieved by providing a receptacle for receiving and containing a liquid or gel-like substance.

The liquid or gel-like substance comprises a substrate and particles therein. Said particles may be cells or microtissues. The cells or microtissues may be naturally comprised in the substance, for example in a whole blood sample or a processed blood sample. The cells or microtissues may also be added to a substrate, which may for example be a hydrogel.

The receptacle comprises a frame member arranged around the periphery of a base member. The frame member and the base member are arranged such that they can be translationally moved in relation to one another. Specifically, in a receptacle positioned on a support surface such that it can contain a liquid, the frame member is adapted to perform an upwards and downwards movement in respect of the base member.

The base member preferably has a flat upper surface extending between the walls of the frame member.

The walls of the frame member are preferably arranged perpendicularly to the xy plane of the base member, respectively its upper surface.

The movement of the frame member is performed in z direction in relation of the xy plane of the base member.

The modular design of the receptacle with its frame member which can be moved up and down in relation to the base of the receptacle, allows to vary the volume of the inner space of the receptacle. By altering the position of the frame member in relation to the base member, the maximum filling volume of the receptacle can be defined. This allows for tailoring the filling volume according to the application and thus provides an increased flexibility. The better control of the filling volume allows for a better adjustment of the receptacle to specific needs.

Preferably, the frame member is arranged to be pushed downwards in relation to a statically disposed base member when the receptacle transitions from its charge state to its discharge state. It was found that the risk or damaging the edges of the solidified tissue sample was significantly reduced when the frame was moved downwards along the static base member. The integrity of the tissue sample is therefore generally improved when the frame is moved in respect of the base, rather than the other way around. It is possible that the shear forces acting on the peripheral edges of the solidified tissue sample are larger when the base member is actively pushed upwardly within the frame, thereby causing some minimal damage along the edges of the sample. However, the damages are considerably less compared to a removal of the sample from a conventional flat receptable.

The modular design of the receptacle renders the removal of the substance after its solidification in the receptacle significantly easier.

While primarily adapted for the formation of solidified biological tissues, the receptacle may also be used for solidification of alternative substances. Such substances may comprise particles. The particles may be patterned in the receptacle, for example by generating a wave pattern in the substance.

Generally, when particles are patterned in a gel-like substance, a solidification step is not necessarily required. However, it is advantageous if the gel-like substance further solidifies, i.e. hardens.

The solidification step of the substance is particularly important in the generation of biological tissue constructs from microtissues or cells dispersed in the substance for example.

In a preferred embodiment of the tissue formation process the solidification is a coagulation of a whole blood sample or of a processed blood sample. The whole blood sample or the processed blood sample may be provided with coagulation promoting and/or coagulation enabling components.

Removing the solidified substance, in particular elastic but fragile tissue construct, without damaging the substance in the process is extremely challenging. This is particularly the case for substances which have the tendency to adhere to the surface of the base member, such as biological tissue constructs. The modular arrangement of the receptacle allows for the frame member to be moved away or totally removed from the base member such that the upper surface of the base member on which the solidified substance is disposed, can be accessed from all sides. It is therefore easier and less risky to remove the solidified substance from the upper surface of the base member without inflicting damage, thus contributing to the reproducibility of results in the fabrication of solidified substance samples.

The receptacle is configured to alternate between a charge state and a discharge state.

The receptacle is in its charge state when the base member, in particular the upper surface of the base member, together with the frame member delimit a space for receiving and containing a liquid substance.

The receptacle is in its discharge state when the base member, in particular the upper surface of the base member, is aligned with or projects beyond a rim of the frame member, such that the substance contained in the receptacle is lifted above said rim of the frame member. In other words, in this state the base member is positioned in alignment with or above the xy plane of the upper rim of the frame member.

The ease of alternation between charge state and discharge state, by a simple translocation of the frame member in respect of the base member, significantly simplify and expedite the manipulation of the device before and after the solidification of a substance in the receptacle. In particular the removal of a solid or gel-like substance from the base member becomes less risky and delicate and can therefore be performed more efficiently.

In one embodiment the receptacle further comprises a support member.

The support member is arranged between the frame member and the acoustic wave generating device. The support member is dimensioned to support the base member as well as the frame member. It may be a plate.

The base member is disposed in an at least laterally fixed position on the support member, i.e. the base member cannot be moved sideways on the surface of the support member. At least when the vibration generating device is activated the base member is fixed to the support member. Support member and base member may be designed such that the base member can be reversibly removed from the support member.

The frame member is movably disposed on the support member such that the receptacle can alternate between its charge state and its discharge state by moving the frame member upwards and downwards in respect of the base member.

The support member further facilitates the handling of the receptacle, in particular the translational movement of the frame. It provides a surface against which the frame member may abut when the receptacle is in its discharge position. It may be provided with biasing means, for example helical springs, which urge the frame member upwardly away from the support member such that the receptacle takes its charge state. The receptacle is thus stabilized in its charge state in the absence of a counterforce exerted on the biasing elements. For the discharge of the substance contained in the receptacle the frame member is pushed downwards against the force of the biasing element.

In a preferred embodiment, the receptacle further comprises one or more interface members for operationally coupling the receptacle to an external vibration generating device. The one or more interface members may be configured to attach the receptacle mechanically and/or magnetically to the vibration generating device.

At least one of the one or more interface members may be comprised in or attached to the base member.

The mechanic and/or magnetic interface between the receptacle and the vibration generating device provides for an integrated system which is specifically designed for wave generation, preferably the generation of waves in the acoustic range, in liquids or gel-like substances and /or patterning of particles in liquids.

Preferably the receptacle is reversibly attached to the vibration generating device during operation of the device, allowing the receptacle to be removed thereafter, for example for cleaning purposes. The mechanical and/or magnetic interface ensures that the receptable is always attached in the same position to the vibration generating device, thereby reducing the risk of variations between different wave generation cycles or between different samples.

In embodiments comprising a support member, at least one of the one or more interface members may be comprised in or attached to the support member. The support member thus operationally couples the receptacle to the vibration generating device.

The invention further provides for a system for generating a wave, for example an acoustic wave, in a liquid or gel-like substance comprising a vibration generating device and the receptacle described herein.

The interface member of the receptacle cooperates with a corresponding connection member of the vibration generating device to operatively couple the two devices. The mechanical and/or magnetic interface couples the receptacle to the vibration generating device in a fixed and robust way, such that the connection is not weakened when exposed vibrations, in particular to vibrations in the sonic frequency range.

The receptacle therefore oscillates with the vibrating portion of the vibration generating device. The mechanical and/or magnetic interface therefore couples the vibration of these two devices, thereby enabling the generation of wave patterns in a liquid substance contained in the receptacle.

In a preferred embodiment the cooperation between the interface member and the connection member is reversible, such that the receptacle may be removed from the vibration generating device when not in use.

The vibration generating device is a vibration actuator arranged to cause controlled vibration of the receptacle.

The vibration actuator may be a mechanic vibration motor, an electromagnetic vibration motor, or a sonic wave generator.

The frequencies of the waves are preferably in the sound wave range.

In one embodiment the wherein the vibration generating device is therefore arranged to oscillate at a frequency range of 20 Hz to 20 kHz, or 20Hz to 1kHz, or 20Hz to 200Hz, or 10kHz to 15kHz.

For generating a wave in a liquid substance contained in a receptacle according to this invention, the receptacle is mechanically and/or magnetically coupled to the vibration generating device through its one or more interface member. Either before its attachment to the vibration generating device or thereafter, a liquid or gel-like substance, for example a whole blood sample, is filled into the receptacle. The vibration generating device is activated to vibrate thereby resulting in the formation of a vibrational wave in the liquid substance contained in the receptacle.

This method can be used to pattern particles contained in the liquid substance, wherein the pattern of the standing or the chaotic acoustic waves in the liquid guide the particles contained therein to defined positions. The particles are thereby dispersed in distinct positions on the basis of the pattern of the wave. The vibrations can also be used to separate particles from each other. This is particularly useful for samples in which particles, such as cells or microtissues, stick together forming an agglomerate.

While the method described herein may generally be used for any processes requiring vibration-induced separation or patterning of particles in a liquid or gel-like substance, it is particularly suited for the generating a biological tissue construct.

As mentioned above, the receptacle is advantageously configured to alternate between its charge state and its discharge state. When in its charge state, a liquid substance containing cells and/or microtissues is first exposed to a vibrational treatment to distribute the cells and/or microtissues throughout the liquid according to the pattern of the waves generated in the substance. Either following the patterning step and/or while subjected to vibration, the substance containing the cells and/or microtissues is incubated for a period of time to allow for solidification of the substance into a gel-like or a solid consistency. The patterned particles are embedded in the solidified substance at the position defined by the wave generated in the substance. The solidified substance may provide a scaffold for the patterned particles.

The incubation period may vary according to the kind of liquid or gel-like substance, respectively the nature and the amount of solidification-promoting or enabling agents which may be provided to the liquid substance. The liquid substance may for example a blood sample, such as whole blood or a processed blood sample, optionally provided with coagulation promoting agents, such as coagulation initiators. The liquid substance may also be a be a hydrogel.

Once the substance has attained the desired degree of solidification following an appropriate incubation period, the frame member is moved in respect of the base member such that the solidified substance is lifted above the frame member. In this position the solidified substance containing the patterned microtissues or cells can be readily removed from the base member of the receptacle. To this end, a suitable tool, for example a separation device as described below, may be used.

The invention further concerns a separation device configured to be used with the receptacle described herein, in order to facilitate the removal of the solidified, i.e. the gel-like or solid substance, from the receptacle, in particular when the receptacle is in its discharge state.

The separation device comprises a filamentous member. Each of the two ends of the filamentous member being fixed to an attachment portion of a holding member. The attachment member is tight, such that it forms a straight line between its attachment points. The attachment portions are arranged such that each of them can be positioned adjacent to the outside of one of two opposed sides of the frame member. In this position the filamentous member spans across the receptacle perpendicularly to the two opposed sides.

The separation device is adapted to remove the solidified substance from the base member when the receptacle is in its discharge state. To this end, the separation device is in position such that the filamentous member contacts the upper surface of the base member.

To separate the solidified substance from the base member, the filamentous member is simply slid across the supper surface, thereby releasing the substance from the surface. The solidified substance does no longer adhere to the base plate and is therefore easy to remove. To improve the stability of this separation device the attachment portions should be resting on a surface, for example the surface of the support member, when the separation device is moved along the receptacle for separating the solidified substance from the base member.

The attachment portions of the separation device are dimensioned such that the filamentous member contacts the upper surface of the base member when the separation member is positioned on a receptacle in its discharge state.

In a preferred embodiment the attachment portions are shaped such that the distance between the filamentous member and a surface on which the attachment members are disposed remains constant when the attachment members are tilted about an axis defined by the filamentous member. In other words, when positioned for separating the solidified substance from the receptacle in its discharge state, the filamentous member will remain in contact with the upper surface of the base member if the holding device is tilted about the filamentous member. By keeping the filamentous fibre in contact with the base plate while sliding it across the receptacle to liberate the solidified substance from the base member regardless of the tilting angle of the separation device, a consistently clean separation of the solidified substance is ensured. The risk of damage to the solidified substance due to tilting of the separation device is thereby avoided.

With respect to what is known in the art, the invention provides a receptacle for forming a solidified tissue sample which can be easily and safely removed from the receptacle in which it was formed. IT therefore allows for the reproducible generation of solidified tissue samples generated *in vitro.*

Moreover, the invention provides for different compatible components of an integrated system for a reproducible generation of solid or gel-like substances with patterned particles, which may serve different purposes. The system is of particular interest for biological tissue generation using waves generated in a substance for patterning of microtissues and/or cells comprised in said substance.

### DEFINITIONS

The term "solidification" as used herein refers to the process of becoming harder or compacter in mass. The term "solidification" includes the hardening of a liquid substance into a gel-like consistency, i.e. a semi-solid consistency, or into a solid consistency, as well as the hardening of a gel-like substance into a solid substance. Coagulation of a blood sample is an example of a solidification process.

The term "processed blood sample" as used herein refers to blood plasma, blood serum, or other fragmentations of blood. A processed blood sample may be obtained through centrifugation of whole blood, centrifugation of clotted blood, or centrifugation of blood comprising anti-coagulants.

The term "upper" refer to a portion of a member or a component which, when the receptacle is disposed on a surface in such a way that it can contain a liquid, faces upwards or which is at a larger distance from the surface on which the receptacle is disposed than the other portions of said member. In analogy, the term "upwards" signifies the perpendicular direction away from said surface.

The term "lower portion" signifies located closer to the surface on which the receptacle is disposed than an upper portion.

The singular "a", "an" and "the" include the plural equivalents unless the context distinctly indicates otherwise.

The terms "comprises" and "contains mean "includes" in a non-limiting sense.

The terms "e.g.", "for example" and "such as" as used herein indicate specific non-limiting examples that fall under a more general category.

### Short description of the drawings

Exemplar embodiments of the invention are disclosed in the description and illustrated by the drawings in which:
Figure 1A depicts a schematic perspective view from above of a first embodiment of a receptacle coupled to a vibration generating device according to this invention;
Figure 1B depicts a perspective view from below of the embodiment of Figure 1A;
Figure 1C is an A-A cross-sectional view of the embodiment shown in Figure 1A showing the receptacle in its charge state;
Figure 2A depicts a schematic perspective view from above of a second embodiment of a receptacle according to this invention, the receptacle is adapted to be coupled to the vibration generating device;
Figure 2B depicts a perspective view from below of the embodiment of Figure 2A;
Figure 2C shows the receptacle of Figure 2A in its discharge state without the protective cover;
Figure 3A shows a B-B cross-section of the receptacle depicted in Figure 2A when in its charge state;
Figure 3B shows a C-C cross-section of the receptacle depicted in Figure 2A when in its charge state;
Figure 3C shows a B-B cross-section of the receptacle depicted in Figure 2A when in its discharge state;
Figure 3D shows a C-C cross-section of the receptacle depicted in Figure 2A when in its discharge state;
Figure 3E shows a perspective view of a B-B cross-section of the receptacle depicted in Figure 2A when in its charge state;
Figure 3F shows a perspective view of a B-B cross-section of the receptacle depicted in Figure 2A when in its discharge state;
Figure 3G shows the view of Figure 3F without the protective cover;
Figure 4A depicts schematically a perspective view of a separation device according to this invention,
Figure 4B is a view from the top of the separation device shown in Figure 4A,
Figure 4C is a lateral view of the separation device shown in Figure 4A,
Figure 5A is an exemplary flow diagram depicting a series of steps for the generation of a solidified substance with patterned particles in the receptacle,
and
Figure 5B is an exemplary flow diagram depicting the process of removing the solidified substance with patterned particles from the receptacle.

### Examples of embodiments of the present invention

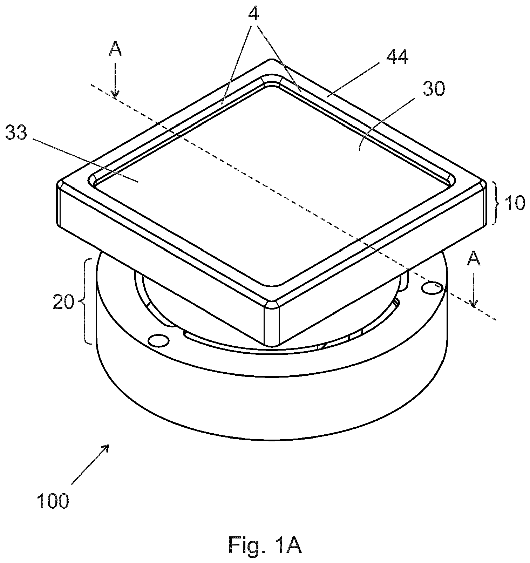

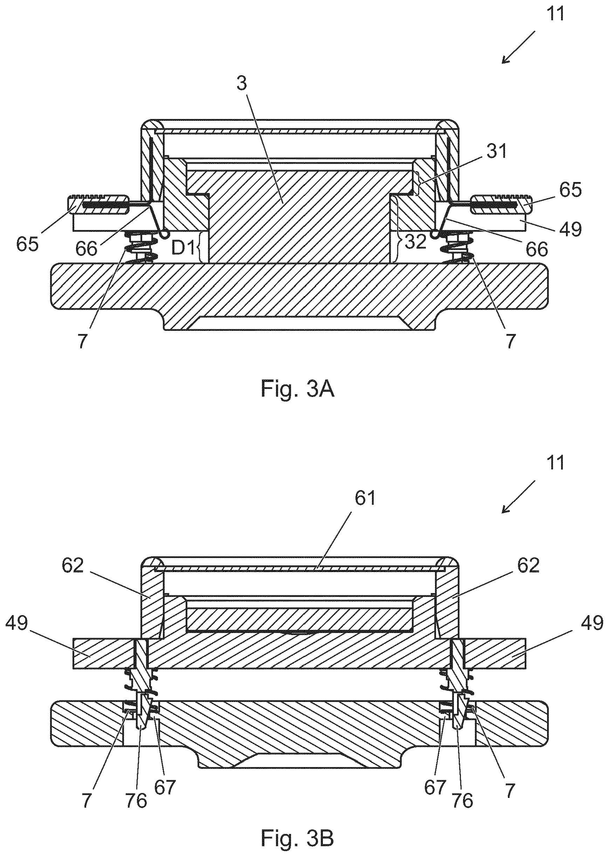

The invention is illustrated on the basis of specific examples shown in the Figures, whereby Figures 1A to 1C show a first example of a receptacle 10 which is coupled to a vibration generating device, which is a vibration motor 20. In Figures 3A to 3G a second preferred embodiment of the receptacle 10 is presented. Both embodiments of the receptacles shown in the Figures are provided with an interface member 12 which is compatible with a corresponding connection member of the vibration generating device 20, which may for example be a vibration motor with a cam mechanism.

In Figure 1A the receptacle 10 is in its charge state, in which the upper surface 33 of a base member 3 together with the side walls of a frame member 4 confine a recess 30 which is adapted to contain a liquid substance. The base member 3 and the frame member 4 can translationally moved in respect of one another. This way, the depth of the recess 30 and therefore the volume of liquid it can hold, may be varied.

It is for example possible to define and temporarily fix different positions of the frame member 4 in respect of the base member 3 to adapt the volume of the recess 30, also referred to as inner volume, of the receptacle 10.

The frame member 4 and the base member 3 thus together confine a recess 30 which is sealed around the periphery of the upper surface 33 of the base member such as to prevent leaking of the liquid medium from the receptacle. The seal between the frame member 4 and the base member 3 does however not prevent the translational movement of these members in respect of each other. To ensure sufficient liquid tightness, a seal element, for example a rubber or silicon seal element, may be suitably arranged between the frame member 4 and the base member 3.

When used for patterning particles dispersed in a liquid substance using vibrations, the upper surface 33 of the base member is preferably flat. A shape with protrusions, depressions or an uneven of curved shape of upper surface 33 could interfere with the formation of the intended distribution of the particles during patterning.

When the upper surface 33 of the base member is aligned with or is lifted above the upper rim 44 of the frame member, the inner volume is zero and the substance which was comprised in the recess is no longer laterally confined. This state of the receptacle is the discharge state, in which a substance, which has solidified in the recess 30 of the receptacle, can now be easily separated from the base member 3 as the lateral delimitations are removed.

The receptacle 10 is coupled to a vibration motor 20 through a mechanical interface consisting of interface members 12 of the base member (not shown) and corresponding connection members (not shown) of the vibration motor 20. It is also possible to couple the receptacle to the vibration generating device through a magnetic interface, or to use a combination of a magnetic and a mechanical interface.

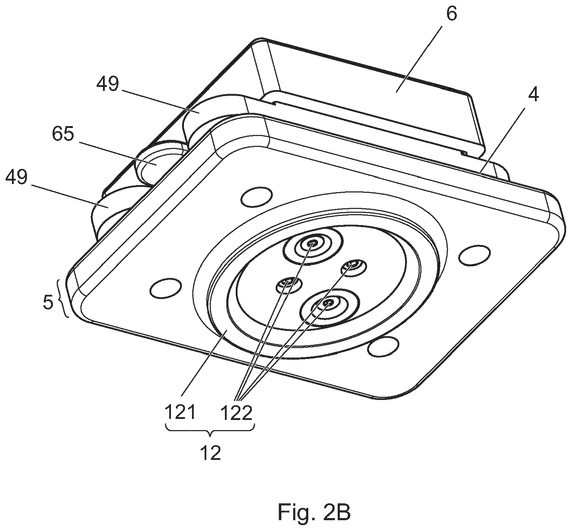

The interface member 12 may for example be a mechanical interface with protrusions and holes configured to engage with a corresponding connection member of the vibration generating device 20 as shown in Figure 2B.

Together, the receptacle 10 and the vibration generating device 10, in the example shown the vibration motor with can mechanism, form an integrated system 100 for generating waves in a liquid. In a preferred embodiment the interface between the receptacle and the vibration generating device is designed for an easy and reversible mounting of the receptacle. This allows for removing the receptacle between operations, for example for cleaning purposes.

When used for patterning particles comprised in the substance, it may be desirable to remove the receptacle from the vibration generating device after the patterning is completed, or after the carrier substance of the particles has solidified, in order to free up the vibration generating device for subsequent receptacles. The workflow of patterning particles in a series of receptacles is thus significantly more efficient.

If the system 100 is used for producing particle patterns in a solid substance, for example a solidified sample comprising microtissues and/or cells for biological tissue formation, the receptacle may be removed from the vibration generating device after the patterning step but before the carrier substance has reached its desired solid or gel-like consistency. The vibration generating device can thus be used again for patterning before the solidification of the substance in the previously coupled receptacle is complete.

A system 100 in which the receptacle may be readily removed also offers the possibility that different embodiments of the receptacle can be used with the same vibration generating device, provided they also comprise an interface member 12 which is compatible with the connection member of the vibration generating device.

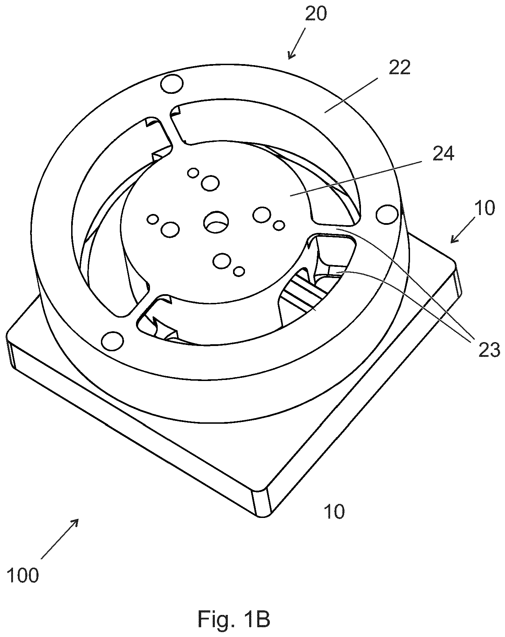

Figure 1B shows a view from below of the system 100 shown in Figure 1A in which the vibration motor 20 is better visible. The illustrated example of the vibration motor 20 comprises an outer annulus which is a static subunit 22 and which contacts the surface on which the vibration motor is disposed. The oscillation of the motor is generated in the centrally located vibration subunit 24 which is connected to the static subunit 22 through several elastic linker 23, which decouples the oscillation of the vibration subunit 24 from the static subunit 22.

The vibration generating device shown in the examples depicted in Figures 1A to 1C is a vibration motor with a cam mechanism. The cam 26 is placed in an aperture 27 in the vibration subunit, causing the subunit to oscillate when set in rotary motion.

In a further embodiment of the vibration motor an oscillation follower, for example a push rod, may be arranged to be lifted by the cam such as to regularly thump against a surface, for example a surface of a support member 5 or the lower surface of the base 3.

The vibration motor may be undergoing a rise-dwell-fall-dwell motion in an oscillating follower cam system.

Other types of actuators which are suitable for generating oscillations are known in the art and may be chosen to generate the acoustic wave in the liquid contained in the receptacle.

The system of this invention is not particularly limited to a specific type of vibration generating device. The device comprises an activator which when activated causes the at least a part of the device to oscillate. Through its firm connection with the oscillating portion of the device the receptacle oscillates with the motor. The firm connection is provided by the engagement of the interface member 12 of the receptacle with a connection member of the vibration generating device.

The vibration generating device may for example be an electromagnetically driven motor. Said motor may comprise a solenoid and plunger mechanism for generating oscillations.

The vibration generating device may be a sonic wave generator.

The vibration generating device may comprise a passive mechanical system for generating oscillations.

The vibration generating device may combine a self-winding rotor mechanism, for example a mechanism as commonly used in automatic mechanical watches, to generate electricity with a piezoelectric quartz crystal as its timing element.

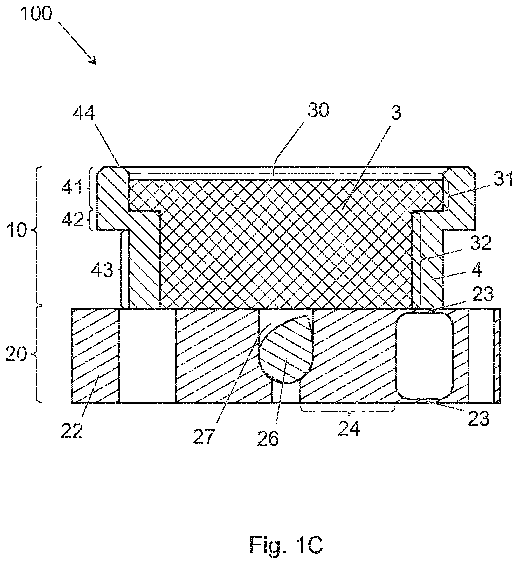

An A-A cross section of the system 100 depicted in Figure 1A is shown in Figure 1C. The receptacle 10 is in its charge state in this Figure. When in its discharge state (not shown) the frame member 4, the base member 3 of the receptable 10 is positioned such that its upper surface aligns with or extends beyond the upper rim 44 of the frame member. In order to move the base member 3 into this position, it must be removed from the vibration generating device 20.

The base member 3 of this embodiment comprises the interface member 12 for coupling the receptacle to the vibration generating device 20. For the receptacle to take its discharge position after solidification of the sample, the base member 3 is moved upwardly at the level or beyond the upper rim 44 of the frame member. This relative movement between frame member 4 and the base member 3 of this embodiment is only possible once the receptacle has been removed from the vibration generating device 20.

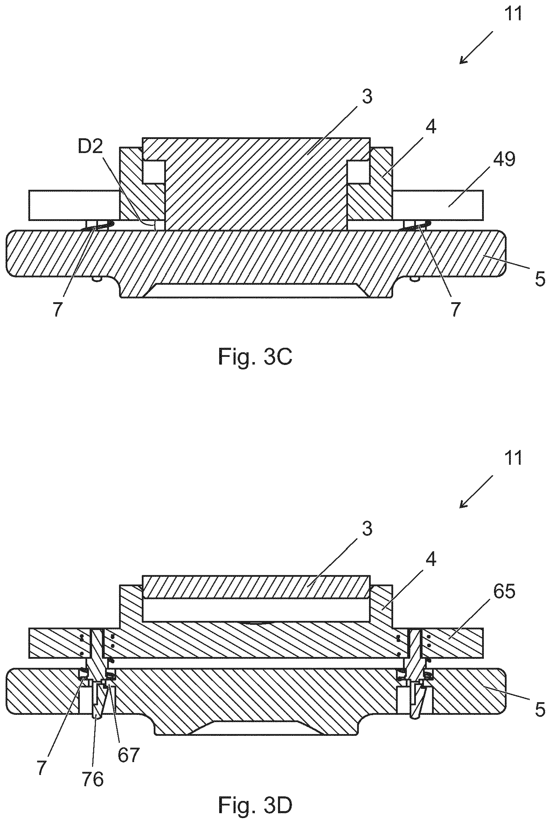

The frame member 4 comprises an upper portion 41 surrounding an upper portion 31 of the base member in the charge state of the receptacle. The frame further comprises a lower portion 43 surrounding a lower portion of the base member 3 in the charge state of the receptacle 11, as shown in Figures 1C and 3D for example.

The frame member 4 is shaped to form a shoulder portion 42 between its upper portion 41 and its lower portion 43, said shoulder portion 42 providing a surface against which the upper portion 31 of the base member can abut in the charge state of the receptacle.

The frame member 4 is movable connected to the support member 5, such that the base member 3 and the frame member 4 can perform a translational movement in respect of each other.

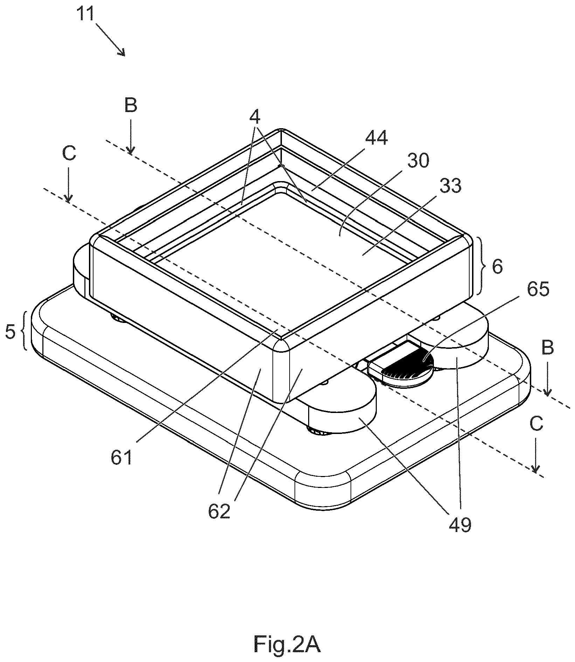

A second embodiment of a receptacle 11 according to this invention is shown in Figure 2A. While this embodiment is shown with an interface member 5, the invention provides also for the same embodiment without an interface member 5, which is equally suitable for incubating a sample during solidification, for example coagulation, and for subsequent safe removal the solidified sample from the receptacle as described below.

In addition to the base member 3, which has a lower portion 31 and an upper portion 32, and the frame member which has a matching upper portion 41 and a matching lower portion 42, the receptacle further comprises a support member 5 onto which the base member is fixed, as shown in Figure 3A and 3C.

The upper portion 32 of the base member forms the upper surface 33. The largest transversal dimension of the upper surface 33 is larger than the largest transversal dimension of a cross-section of the lower portion 31 upper portion which is parallel to said upper surface 33. The upper portion 32 projects laterally over the lower portion 31.

As illustrated in Figure 2A, the upper surface 33 of the base member may be square shaped. The shape is however not particularly limited to a square or a rectangular shape. Other shapes, for example disc or an ellipse, are also possible. The frame is shaped to extend upwardly from the perimeter of the upper surface. The inner wall of the frame may extend perpendicularly to the base plate. It may be curved or angled outwardly towards the upper rim 44 of the frame.

Similar to the first embodiment of the receptacle shown in Figures 1A to 1C, the frame member 4 of this embodiment comprises an upper portion 41, a shoulder 42 and a lower portion 43.

Different from the first embodiment, the lower portion only surrounds an upper section of the lower portion 32 of the base member, such that the lower rim of the frame member 4 is placed at a distance D1 from a surface on which the receptacle is disposed, when the receptacle 11 is in its charge state, as shown in Figure 3A for example.

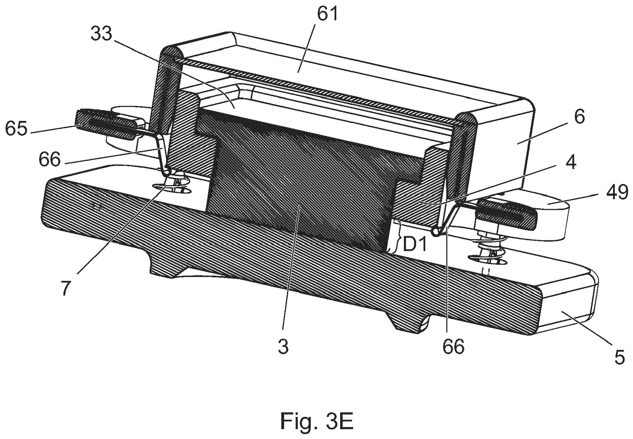

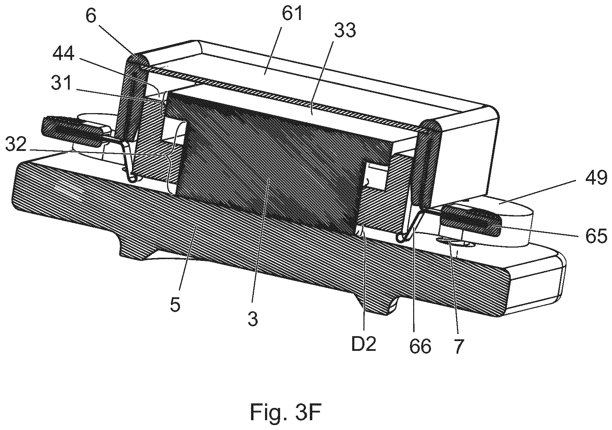

The frame member 4 is movably connected to the support member 5, such that it can perform a translational movement in relation to the base member 3. The distance D1 enables the frame member 4 to be pushed downwards in respect of the base member 3 to change the state of the receptacle from the charge state to its discharge state. The distance D2 between the lower rim of the frame member 4 and the support member 5 is therefore reduced when in the discharge state of the receptacle compared to the distance D1 in the charge state, as exemplified in Figures 3A and 3E depicting the receptacle 11 in its charge state compared to Figures 3C and 3F depicting the receptacle 11 in its discharge state. The distance D2 may be zero, in which case the frame member 4 abuts the support member 5 in the discharge state.

Embodiments in which the frame member 4 is actively pushed downwards in relation to the base member 3 generally produce solidified samples of a better integrity along their periphery than embodiments in which the base member 3 is pushed upwards in relation to the frame member 4.

In this second embodiment, as shown in Figure 2A, suitable shaped protrusions 49 extending laterally from the frame member 4 are arranged to facilitate exerting a downward force onto the frame member 4 when the receptacle 11 is moved into its discharge state.

In a preferred embodiment the frame member 4 is urged upwardly by suitably arranged biasing means 7, for example a helical spring, such that the shoulder 42 abuts the upper portion 31 of the base member.

The receptacle shown in Figures 2A and 2B further comprises a protective cover 6 with a cover plate 61 and side walls 62 which prevent spillage of the oscillating liquid. The protective cover 6 protects the substance in the receptacle from undesirable external influences, including contamination.

The protective cover is reversibly attached to the frame member 4 of the receptacle by suitable fastening means 66. In the depicted embodiments the fastening means comprise a clip element 66 for fastening the protective cover to the frame lower rim of the frame member. The clip may be fastened and removed using a suitable positioned handling member 65.

In Figure 2B, which is a view from below of the receptacle presented in Figure 2A, the interface member 12, which is comprised in the support member 5 of this embodiment is visible.

The interface member 12 depicted here comprises a series of structural elements 122, 121, including an annular protrusion 121 from the surface of the support member 5 as well a series of holes 122 which are arranged to cooperate with corresponding structural elements of matching shape which are arranged on the surface of the vibration generating device 20. These corresponding members are comprised in the connection member (not shown) of the vibration generating device 20. Through their engagement the interface member 12 and the connection member serve to provide mechanical stability for the receptacle during vibration of the vibration generating device 20.

Compared to single or a series of distinct fixation points, annular engaging structures provide for an improved stability of the receptacle in respect of the vibrating motor and contributes to a robust connection during oscillation cycles.

In embodiments where the vibration generating device is a vibration motor, the annular structures are preferably centred around the vibration generating element of the vibration motor, for example a cam or a oscillating follower.

For optimized stability between a receptacle 10, 11 and a vibration generating device 20 such compatibly engaging annular structures, for example an annular protrusion121 as shown in Figure 2B and a matching depression in the connection member of the vibration generating device 20, are combined with one or more holes 122 which engage with matching protrusions. The holes 122 preferably have a greater depth than a depression accommodating the annular protrusion. The holes may be spread inside the region delimited by the annular portion as shown in Figure 2B.

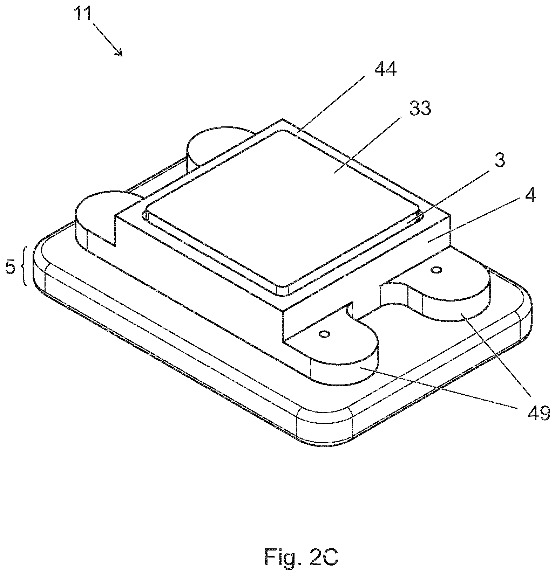

Figure 2C shows the receptacle of Figures 2A and 2B without the protective cover 6 in its discharge state. The upper surface 33 of the base member 3 is raised above the upper rim 44 of the frame member 4 in this state. The frame member 44 is pushed downwards in direction of the support member 6 to let the base member 3 protrude above its upper rim 44. The lateral protrusions 49 may be used to provide the downwards force. This is particularly useful, if the frame member 4 is pushed towards the support member 6 against the biasing force of a biasing means 7, as shown in Figures 3A and to 3D.

Figure 3A, which represents a B-B cross section of the receptacle shown in Figure 2A when in its charge state, illustrated the frame member 4 being urged upwardly by two helical springs 7 so its inward facing shoulder 42 contacts the upper portion 31 of the base member 3. The abutment of the shoulder against this upper portion 31 prevents the frame member 4 from being pushed upwardly any further. The abutment thereby defines the maximum inner volume of the recess 30 formed by the inner walls of the frame member 4 and the upper surface 33 of the base member 3. In this example, the receptacle 10 is provided with a protective cover 6 held in place by lateral clips 66. The clips can be loosened by pulling the handling element 65 of the clip 66 away from the receptacle in an outwards direction.

Figure 3B depicts another cross-section C-C of the same receptacle at the hight of engagement elements 67. The engagement elements 67 attach the frame member 4 to the support member 5, whilst allowing a translational movement of the frame member 4 in relation to the base member 3 when in an open position as shown in the cross-sectional view of Figure 3B. The engagement elements 76 are shaped to snap into a locked position with corresponding structural elements 67 when the receptacle is in its discharge state.

When the frame member 4 is urged downwards against the force of the helical springs 7, the engagement elements 76 snap into the corresponding structural elements 67, in this case protrusions, of the frame to maintain the frame member 4 in its lowered position in respect to the base member 3 as shown in Figures 3C and 3D.

Figure 3C corresponds to the B-B cross sectional view of Figure 3A and Figure 3D corresponds to the C-C cross sectional view of Figure 3B, whereby the receptacle is in its discharge state in both Figures.

As a result, the discharge state of the receptacle is sufficiently stable to permit for removal of a substance disposed on the base member 3 without actively applying continued downward force onto the frame member 4.

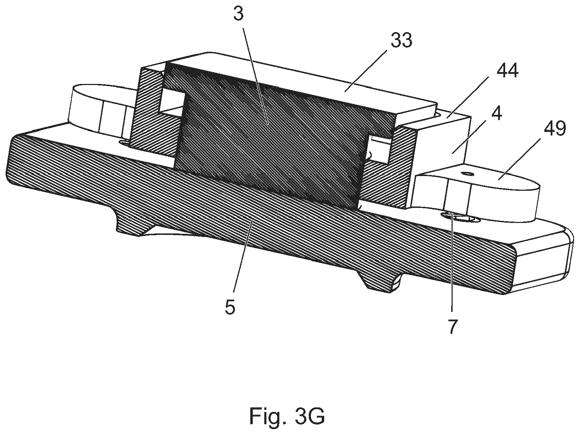

Figures 3E to 3G show perspective cross-sectional views of the receptacle in its charge state (Figure 3E) or in its discharge state (Figures 3F and 3G), to further illustrate the mechanism. The protective cover 6 which is reversibly fixed onto the frame member 4 of the receptacle in Figures 3E and 3F is removed in Figure 3G.

Figure 3G represents the state of the receptacle when a solidified substance disposed on the upper surface 33 of the base member 3 is made accessible for its removal from the receptacle.

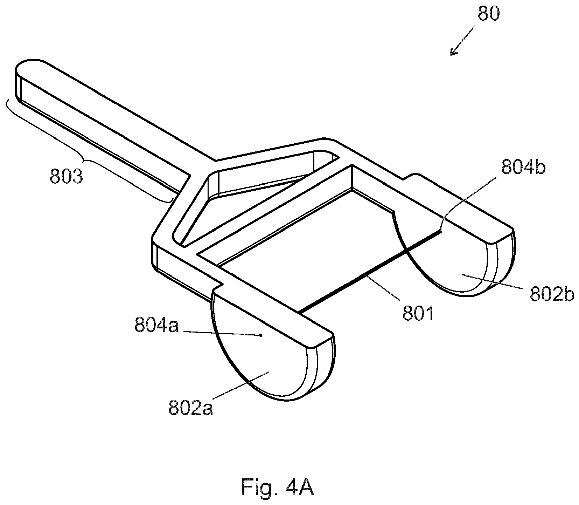

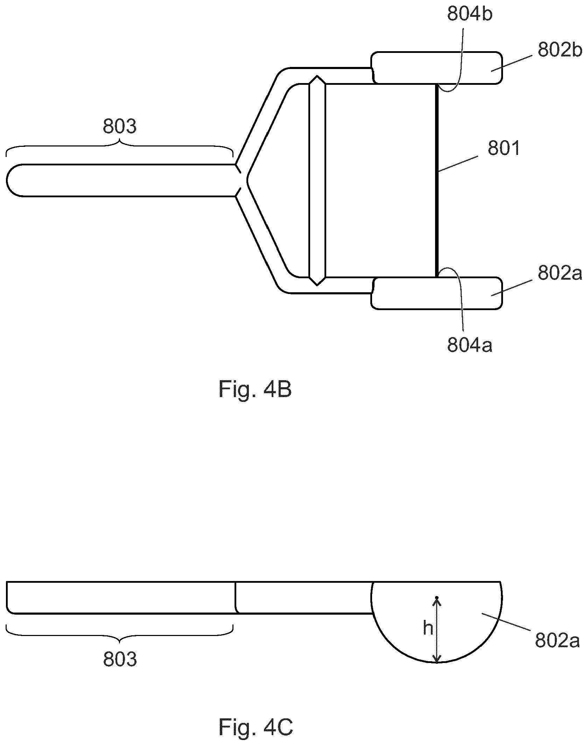

To facilitate the removal of solidified substances which have the tendency to stick to a surface, in particular gel-like substances or biological tissues, from the upper surface 33 of the base member 3, a suitable dimensioned separation device 80 as schematically presented in Figures 4A to 4C may be used.

The separation device shown in these figures comprises a filamentous member 801 which is spanned between two lateral attachment portions 802a, 802b of the separation device. Each end of the filamentous member 801 is attached to an attachment portion 802a, 802b in an attachment point 804a, 804b.

A holding member 803 is provided to enable a user to hold and handle the device.

To liberate a solidified substance provided on the upper surface 33 of the base member 3, the receptacle 10, 11 in its discharge state is positioned between the attachment portions, such that the filamentous member 801 contacts the edge of the upper surface 33 of the base member 3. In other words, each of the attachment members 802a, 802b is positioned adjacent to the outside of a portion of one of two opposed sides of the frame member such that the filamentous member 801 spans across the receptacle 10, 11 in a straight line perpendicular to said two opposed sides, touching the edge of the upper surface 33 of the base member in its discharge state.

The hight h between the attachment points 804a, 804b for the filamentous member 801 in the attachment portions 802a, 802b and the surface of the attachment portions 802a, 802b which rests on a surface when the separation device is positioned for liberating the solidified substance from the receptacle, is therefore advantageously adjusted according to the distance between the upper surface 33 of the base member 3 and this same surface on which the attachment portion rests.

In a preferred embodiment, the attachment portions 802a, 802b are shaped as a disc or a portion of a disc, with the attachment points 804a, 804b being located at the centre of said discs or portions of discs. This embodiment provides the advantage, that the hight h remains the same independent of the tilt angle of the device, such that the filamentous member 801 remains in contact with the upper surface 33 of the base member when in position independent of the tilt angle of the separation device 80.

When in starting position for separating the solidified substance from the upper surface 33, the filamentous member 801 contacts the edge of the surface member, such that it slides between the solidified substance and the upper surface 33 when the separation device is moved translationally along the length or width of the receptacle. Any adhesion between the solidified substance and the upper surface 33 is thereby mechanically disrupted, such that the removal of the solidified substance from the upper surface 33 of the base member is facilitated.

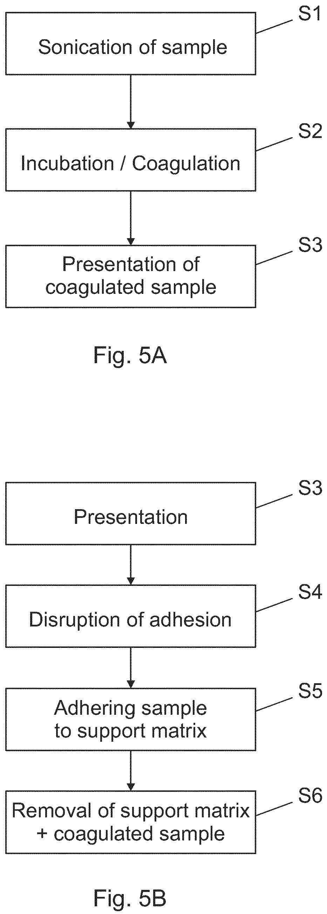

An exemplary process of generating a tissue sample form blood or processed blood using the device of this invention is presented in the flow diagram of Figure 5A. It is also possible to use hydrogel as a carrier substance for the cells or microtissues instead of blood or processed blood.

In a first step S1 the sample, which may be a whole blood sample, a processed blood sample or a hydrogel sample comprising biological cells or microtissues, is sonicated using the receptacle connected ton vibration generating device according to this invention.

In a second step S2, the sample is resting in the receptacle to allow for coagulation of the blood sample or processed blood sample, respectively for solidification of the hydrogel matrix.

Once the sample in the receptacle 10, 11 has attained its desired solidified consistency, the base member 3 is raised in relation to the frame member 4 of the receptacle 10, 11 in step S3, such that the solidified sample is presented on the upper surface 33 of the receptacle at the hight of the upper rim 44 of the frame or above.

To remove the coagulated blood sample or the solidified hydrogel sample, the adhesion of the sample to the upper surface 33 of the base member is disrupted in step S4, as shown in Figure 5B which depicts the removal process of the solidified sample.

The disruption of the adhesion is preferably performed using the separation device 80 of this invention as described above.

In a subsequent step S5 the sample is adhered to a support matrix, which is layered on the upward facing surface of the coagulated or solidified sample. The support matrix may for example be a sheet of silicon such than a dressing, a gauze or a biomaterial such than a collagen or fish-skin. The support matrix can also be an autologous tissue, for example a skin graft which adheres to the sample upon contacting same.

Once the entire surface of the sample is adhered to the support matrix, the support matrix together with the solidified sample, for example a blood clot, is peeled off the upper surface 33 of the base member, such that the solidified sample is completely removed from the receptacle 10, 11.

Using the support matrix the solidified sample, e.g. the coagulated blood sample, may thus be transferred to another surface, for example an open wound.

## Claims

1. Receptacle (10, 11) for forming a solidified biological tissue sample, the receptacle comprising a frame member (4) arranged around the periphery of a base member (3), such that said frame member (4) and said base member (3) can perform a translational movement in relation to one another, wherein the receptacle (10, 11) takes a charge state when the base member (3) together with the frame member (4) delimit a space for receiving and containing a liquid or a gel-like substance, and wherein the receptacle (10, 11) takes a discharge state when the base member (3) is aligned with or projects beyond a rim (44) of the frame member (4), such that the substance contained in the receptacle (10, 11) is lifted above said rim (44) of the frame member (4).

2. The receptacle of claim 1, wherein the frame member (4) is arranged to be moved downwards in relation to a statically disposed base member (3) when the receptacle transitions from its charge state to its discharge state.

3. The receptacle of claim 1 or 2, further comprising a support member (5),
- wherein the base member (3) is disposed in a fixed position on the support member (5), and
- wherein the frame member (4) is movably disposed on the support member (5) such that the receptacle (11) can alternate between its charge state and its discharge state.

4. The receptacle of any of claims 1 to 3, further comprising one or more interface members (12) for operationally coupling the receptacle (10, 11) to a vibration generating device (20), such that waves are formed in the liquid and/or gel-like substance contained in the receptacle when the vibration generating device is activated.

5. The receptacle of any of claims 1 to 4, wherein the one or more interface members (12) couple the receptacle (10, 11) mechanically and/or magnetically to the vibration generating device (20).

6. A separation device (80) configured to facilitate the separation of a gel-like or a solid substance from the base member (3) of the receptacle (10, 11) of any of claims 1 to 5 comprising a filamentous member (801), each of the two ends of said filamentous member being fixed to an attachment portion (802a, 802b) connected to a holding member (803), said attachment portions being arranged to each be positioned adjacent to the outside of one of two opposed sides of the frame member such that the filamentous member (801) spans across the receptacle (10, 11) in a straight line perpendicular to said two opposed sides.

7. The separation device of claim 6, wherein the attachment portions (802a, 802b) are dimensioned such that the filamentous member (801) fixed thereon contacts an upper surface (33) of the base member (3) when the separation member (80) is positioned on a receptacle (10, 11) in its discharge state.

8. A set of the receptacle (10, 11) of any of claims 1 to 5 and of the separation device (80) of claim 6 or 7.

9. A system for generating a wave in a liquid or gel-like substance comprising a vibration generating device (20) and the receptacle (10, 11) of claim 4 or 5, wherein the one or more interface members (12) of the receptacle cooperate with one or more corresponding connection members of the vibration generating device (20), such as to cause the receptacle (10, 11) to oscillate with the vibration generated by the vibration generating device (20) thereby causing the generation of waves in a liquid or gel-like substance contained in the receptacle (10, 11).

10. The system of claim 9, wherein the vibration generating device (20) is a mechanic vibration actuator or an electromagnetic vibration actuator.

11. The system of claim 9 to 10, wherein the vibration generating device is arranged to produce waves having a frequency range of 20 Hz to 20 kHz, or of 20 Hz to 1kHz, or of 20Hz to 200Hz, or of 10kHz to 15kHz in a liquid or gel-like substance contained in the receptacle.

12. Use of the receptacle of any of claims 1 to 5 or of the system of any of claims 9 to 11 for the generation of biological tissue from cells and/or from microtissues dispersed in a liquid or gel-like substance.

13. Use according to claim 12, wherein the biological tissue is generated using a whole blood sample, using a processed blood sample, or using a hydrogel.

14. Method for generating a wave in a liquid substance comprising
- providing a receptacle (10, 11) for receiving and containing a liquid substance, the receptacle comprising a frame member (4) arranged around the periphery of a base member (3) such that said frame member (4) and said base member (3) can perform a translational movement in relation to one another, further comprising one or more interface members (12) for operationally coupling the receptacle (10, 11) to a vibration generating device (20),
- mechanically and/or magnetically coupling the receptacle (10, 11) through its one or more interface member (12) to the vibration generating device (20,
- providing a liquid or gel-like substance to the receptacle (10, 11),
- activating the vibration generating device (20) such as to cause the receptacle to oscillate, thereby inducing the formation of a wave in the liquid or gel-like substance contained in the receptacle (10, 11),
wherein the step of coupling the receptacle (10, 11) to the vibration generating device (20) and the step of providing the liquid or gel-like substance to the receptacle may be performed in reversed order.

15. Method for patterning of particles comprising providing a liquid or gel-like substance containing the particles to be patterned and performing the method of claim 14, thereby producing a wave in the liquid or gel-like substance which directs the particles to distinct positions defined by said wave.

16. Method for generating a biological tissue construct, the method comprising performing the method of claim 15, wherein the particles contained in the liquid or gel-like substance are biological cells and/or microtissues, wherein the receptacle (10, 11) is configured to take a charge state when the base member (3) together with the frame member (4) delimit a space for receiving and containing a liquid or gel-like substance, and wherein the receptacle (10, 11) takes a discharge state when the base member (3) is aligned with or projects beyond an upper rim (44) of the frame member (4), further comprising the steps of
- incubating the liquid or gel-like substance containing the patterned cells and/or microtissues for a period of time to allow for solidification of the substance contained in the receptacle (10, 11) into a gel-like or a solid consistency, and
- following the incubation period, moving the frame member (4) in relation to the base member (3) such that the receptacle (10, 11) takes its discharge state, thereby lifting the solidified substance containing the patterned cells and/or microtissues above the frame member (4).
